# EUROPEAN PATENT APPLICATION

(11) **EP 4 617 834 A2**
(43) Date of publication of application: **17.09.2025**
(21) Application number: 25192599.6
(22) Date of filing: 29.07.2025
(51) Int. Cl.: G06F 3/01, G06F 3/0362, A61B 5/24, G05G 1/10, G06F 1/16, H01H 19/00, H03K 17/97

(54) **ELECTRONIC DEVICES WITH CROWN ASSEMBLIES HAVING A MAGNETIC SENSOR**

(30) Priority: 30.07.2024 US 202418789457
(71) Applicant: Apple Inc., Cupertino, CA 95014 (US)
(72) Inventor: MOORTHY, Sriram, Cupertino, 95014 (US); LIN, Tsu-Hui, Cupertino, 95014 (US); DASHEVSKY, David D, Cupertino, 95014 (US); ATURA BUSHNELL, Tyler S., Cupertino, 95014 (US); SWEET, Stephen N., Cupertino, 95014 (US); PIERRE, Joseph J., Cupertino, 95014 (US); DALMADY, Cynthia, Cupertino, 95014 (US)
(74) Representative: Rooney, John-Paul

(57) **Abstract**

An electronic watch (200) described herein may include an external crown (204) that is positioned along an external side (202a) of a wall segment of a housing (202) of the electronic device. The crown (204) may be configured to rotate in response to a user input and may be coupled to a magnetic member (226) that can rotate with the crown (204). A magnetic sensing system is positioned within an internal volume of the device (200), defined by the housing (202), and may be configured to detect a change in magnetic field due to the rotation of the magnetic member through the wall segment of the housing (202).

## Description

### TECHNICAL FIELD

The subject matter of this disclosure relates generally to electronic devices, and more particularly, to electronic devices with crowns having magnetic sensors.

### BACKGROUND

Electronic devices such as mobile phones, tablet computers, wearable devices, and the like may include input systems, such as buttons, crowns, dials, and the like, which can detect a variety of inputs or other signals. For example, a watch or other electronic device may include a crown that can be rotated and/or pushed in order to provide inputs to the device. Such input systems may be used in various combinations to provide input functionality to a device.

### SUMMARY

An electronic watch, such as described herein, may include a display assembly, a housing, a crown, and a magnetic sensing system. The display assembly may include a display stack and a cover positioned over the display stack. The cover may define a front surface of the electronic watch. The housing may define an exterior side of the electronic watch, a recess along the exterior side, an internal volume, and a wall segment defining an end of the recess and fluidly isolating an external environment from the internal volume. The crown may be positioned along the exterior side of the electronic watch. The crown may include a cap configured to rotate in response to a rotational input, a shaft extending from the cap and positioned within the recess, the shaft configured to rotate in response to the rotational input, and a magnetic member positioned within the recess and coupled to the shaft, the magnetic member configured to rotate in response to the rotational input and to produce a change in a magnetic field that propagates through the wall segment. The magnetic sensing system may be positioned within the internal volume and may be configured to detect the rotational input based at least in part on a change in the magnetic field due to rotation of the magnetic member.

In some cases, the cap, the shaft, and the magnetic member are configured to translate towards the wall segment in response to a translational input, and the magnetic sensing system is configured to detect the translational input based at least in part on a change in the magnetic field due to the translation of the magnetic member. According to some embodiments, the electronic watch further includes a dome switch coupled to the wall segment and a conductive member. The dome switch may be positioned within the recess and opposite the internal volume and may be configured to bias the shaft away from the wall segment. The conductive member may be positioned between the shaft and the dome switch and may be conductively coupled to the wall segment. In some cases, the conductive member is conductively coupled to the cap, the cap defines a user input surface configured to receive an electrocardiogram (ECG) signal from a user, and the electronic watch further includes a processing system conductively coupled to the wall segment and configured to detect the ECG signal via the wall segment.

In some embodiments, the housing has a main body defining an opening extending from the exterior side, and a bucket structure positioned within the opening and configured to fluidly isolate the external environment from the internal volume, the bucket structure defining the wall segment. The recess may be defined by the opening and the bucket structure. In some cases, the wall segment is formed from titanium. In some examples, the magnetic sensing system includes a first tunneling magnetoresistance (TMR) sensor positioned at a first orientation and within the internal volume, and a second TMR sensor positioned at a second orientation different from the first orientation and within the internal volume.

In some examples described herein, an electronic device includes a housing, a crown, and a magnetic sensing system. The housing may be formed from a metal material and include a sidewall and at least partially defining an internal volume of the electronic device. The crown may have a cap and a magnetic member. The cap may be positioned outside of the internal volume and along an exterior side of the sidewall, the cap may be configured to rotate in response to a user input. The magnetic member may be coupled to the cap and positioned outside of the internal volume; the magnetic member may be configured to rotate in response to the user input. The magnetic sensing system may be positioned within the internal volume of the electronic device and along an interior side of the sidewall opposite the exterior side, the magnetic sensing system may be configured to detect the user input based at least in part on a change in a magnetic field due to the rotation of the magnetic member.

In some examples, the housing also defines an external portion, a hole extending through the external portion, and a secondary volume fluidly coupled to an external environment and fluidly isolated from the internal volume, the secondary volume defined by the sidewall and the external portion. In some cases, the crown extends at least partially through the hole and the magnetic member is positioned within the secondary volume. In some embodiments, the user input is a first user input. In some examples, the electronic device includes a dome switch positioned within the secondary volume and coupled to the sidewall. The dome switch may be configured to bias the crown away from the sidewall and collapse in response to a second user input.

In some cases, the housing includes an external protrusion extending from the sidewall. The crown may be coupled to the external protrusion and the magnetic member may be positioned radially outward of the external protrusion. The external protrusion may be configured to transmit an electrocardiogram (ECG) signal from a user input surface to a flexible circuit positioned within the internal volume. In some cases, also includes a haptic actuator positioned within the internal volume of the electronic device and the electronic device may be configured to output a haptic response via the haptic actuator in response to the user input. The electronic device may further include a magnetic shielding member at least partially surrounding the magnetic sensing system, the magnetic shielding member may be configured to magnetically shield the magnetic sensing system from the haptic actuator.

According to some embodiments described herein, an electronic device may include a housing, a crown, and a magnetic sensing system. The housing may include a metal sidewall which may define an exterior surface and an interior surface opposite the exterior surface. The crown may be positioned along the exterior surface. The crown may include a cap configured to rotate in response to a rotational input, and a magnetic member. The magnetic member may be configured to rotate with the cap in response to the rotational input, and propagate a magnetic field through the metal sidewall. The magnetic sensing system may be configured to detect, through the metal sidewall, a change in the magnetic field due to rotation of the magnetic member.

In some cases, the crown further includes a shaft extending from the cap. The magnetic member may be coupled to the shaft. The magnetic member may include a north polarity and a south polarity, the north and south polarities radially opposed about a rotational axis of the crown. In some embodiments, the crown includes a rotating assembly configured to rotate in response to the rotational input. The rotating assembly may include the cap and the magnetic member. In some cases, an entirety of the rotating assembly is external with respect to the exterior surface.

In some examples, the magnetic sensing system includes a first tunneling magnetoresistance (TMR) sensor positioned at a first orientation along the interior surface, and a second TMR sensor positioned at a second orientation different from the first orientation, the second TMR sensor positioned along the interior surface, the magnetic sensing system may be configured to detect a direction of rotation of the rotational input.

In some cases, the magnetic member is a first magnetic member and the crown further includes a second magnetic member. The first and second magnetic members may be positioned along a periphery of the cap. In some examples, the housing is a monolithic structure.

### BRIEF DESCRIPTION OF THE DRAWINGS

Reference will now be made to representative embodiments illustrated in the accompanying figures. It should be understood that the following descriptions are not intended to limit this disclosure to one included embodiment. To the contrary, the disclosure provided herein is intended to cover alternatives, modifications, and equivalents as may be included within the spirit and scope of the described embodiments, and as defined by the appended claims.
FIG. 1 shows an example of an electronic device including a crown, such as described herein.
FIGs. 2A-2B show cross-sectional views of an example crown, such as described herein, along line 2A-2A of FIG. 1.
FIGs. 3A-3B show cross-sectional views of an example crown, such as described herein, along line 3A-3A of FIG. 1.
FIGs. 4A-4B show cross-sectional views of an example crown, such as described herein, along line 3A-3A of FIG. 1.
FIG. 5A shows a perspective view of an example crown, such as described herein.
FIGs. 5B-5D show detail views of components of the crown from FIG. 5A.
FIG. 6 shows example headphones which may incorporate a crown, such as described herein.
FIG. 7 shows an example virtual reality device which may incorporate a crown, such as described herein.
FIG. 8 shows example components of the electronic device.

The use of the same or similar reference numerals in different figures indicates similar, related, or identical items.

The use of cross-hatching or shading in the accompanying figures is generally provided to clarify the boundaries between adjacent elements and also to facilitate legibility of the figures. Accordingly, neither the presence nor the absence of cross-hatching or shading conveys or indicates any preference or requirement for particular materials, material properties, element proportions, element dimensions, commonalities of similarly illustrated elements, or any other characteristic, attribute, or property for any element illustrated in the accompanying figures.

Additionally, it should be understood that the proportions and dimensions (either relative or absolute) of the various features and elements (and collections and groupings thereof) and the boundaries, separations, and positional relationships presented therebetween, are provided in the accompanying figures merely to facilitate an understanding of the various embodiments described herein and, accordingly, may not necessarily be presented or illustrated to scale, and are not intended to indicate any preference or requirement for an illustrated embodiment to the exclusion of embodiments described with reference thereto.

### DETAILED DESCRIPTION

Embodiments described herein relate to electronic devices. In particular, embodiments described herein are directed to electronic devices with rotational input members, such as a crown. The electronic device is configured to detect a rotation and/or a direction of rotation of the crown (and optionally a translation of a crown) using magnetic sensors. In some configurations described herein, all of the rotating components of the crowns are external with respect to a housing of the electronic device. The rotation (and/or translation) of the crown may be detected through a sidewall of the housing using a magnetic sensing system positioned internally with respect to the electronic device.

Electronic devices, such as electronic watches, headphones, virtual headsets, and the like, often include rotary input controls, such as crowns. These crowns are generally configured to receive a variety of user inputs, such as rotational inputs, press inputs (e.g., translational inputs or force-based inputs), and touch inputs. These inputs allow the user to navigate through various menus and/or select options within a user interface, or otherwise control different operations of a device. To receive rotational inputs, crowns generally include rotating shafts which extend through a housing of the electronic device and into the internal volume of the device. However, this structure may introduce leak paths which can also allow the ingress of water and/or other debris into the internal volume of the electronic device.

In some examples described herein, an electronic device includes a crown having a magnetic member that is configured to produce a changing or variable magnetic field when the crown is rotated. This change in magnetic field is detectable by a magnetic sensing system that is positioned in an internal volume of the device. In some configurations, the rotating components or rotating assemblies of the crown may be entirely external with respect to a housing of the electronic device. The magnetic sensing system of the electronic device is positioned such that a rotation of an external crown can be detected through a solid wall. Because the magnetic sensor can sense the rotation through the wall, all of the rotating components of the crown may be positioned external to the housing, obviating the need for through-holes or other openings in the housing to allow crown shafts or other rotating components to enter into the interior volume of the device. Accordingly, seals and other components used to prevent liquid ingress from an external environment into the internal volume of the electronic device can be reduced and/or eliminated-thereby increasing the integrity of the environmental sealing of the device, reducing the complexity and parts which are part of sealing assemblies of the crown, and optionally reducing the footprint of the crown input system.

In some examples, the electronic device may be an electronic watch including a display assembly with a cover that defines a front surface of the watch. A housing of the watch may surround the display assembly and define a side exterior surface of the device. The housing may define a general external boundary of the watch and an internal volume, and the internal volume may be protected from an external environment (e.g., fluidly isolated) by the housing. Generally, the internal volume may include electronic components of the device, such as batteries, processors, sensors, and other components. The housing may include a recessed portion configured to retain an external crown. This recessed portion can be separated from the internal volume by one or more wall portions of the housing.

In some cases, the crown may include a cap that defines a user input surface and which is configured to rotate in response to user inputs. The crown may also include a shaft that extends from the cap and which also rotates in response to user inputs. A magnetic member is coupled to the crown and configured such that a rotation of the crown generates a change in a magnetic field. For example, as a magnetic material rotates, a strength of a magnetic field (e.g., a change) may be detected by the magnetic sensor and correlated to an angle of rotation. In some cases, more than one magnetic sensor positioned at different orientations may be configured to measure a direction of rotation. A magnetic sensing system may be positioned on an opposite side of a sidewall of the housing, within the internal volume. Thus, the magnetic sensing system is positioned internally while the crown is positioned externally. Through the sidewall, the magnetic sensing system may be configured to detect this change in the magnetic field due to the rotation of the crown. In this configuration, the crown (along with all its rotating components) may be fully external with respect to an internal volume of the device (defined in part by the housing). This structure reduces the risk of water ingress into the internal volume and potentially reduces the overall size or footprint of the crown.

In some cases, the magnetic sensing system may include one or more tunneling magnetoresistance (TMR) sensors, which may be positioned at different orientations to detect a direction of rotation of the crown (e.g. based on the manner in which the magnetic field changes and/or moves). The magnetic sensing system may be configured to detect a press input via the same TMR sensors. For example, a magnitude of the magnetic field may increase as the magnetic members translate towards the sidewall of the housing (or a characteristic of the magnetic field may otherwise change in a manner indicative of a translation of the crown).

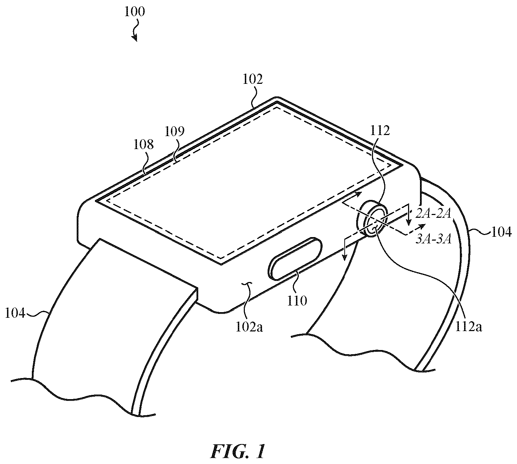

These foregoing and other embodiments are discussed below with reference to FIGs. 1-8. However, those skilled in the art will readily appreciate that the detailed description given herein with respect to these figures is for explanation only and should not be construed as limiting.

FIG. 1A depicts a perspective view of an example electronic device 100 (or device 100) which may include a crown and a magnetic sensing system that detects movement of the crown, such as described herein. In some cases, the device 100 is a watch. In other cases, the crown may be incorporated into electronic devices such as phones, tablets, laptops, AR/VR headsets, headphones, digital media players, and the like.

The device 100 may include a housing 102. The housing 102 may define an internal volume of the device 100. At the internal volume, the device 100 be configured to house internal components of the device, including batteries, processors, sensors, and the like. In some cases, the housing is formed from a metal, such as titanium, aluminum, non-ferromagnetic metals, non-ferromagnetic steels, austenitic steels, or any alloys or combination of materials, as may be known to one of skill in the art. In some cases, the material may be a paramagnetic material. In some examples, the housing is formed from ceramic, glass, polymers, and the like. The housing 102 may be a monolithic piece or may assembled from multiple parts using any suitable method. The housing may define recessed portions, openings, blind holes, and/or coupling features that receive and/or secure user input controls and/or electronic components to the device 100.

A display assembly may be positioned within the housing 102. The display assembly may include a cover 108 and a display stack 109. Generally, the cover 108 may be positioned over the display stack 109. The cover may be glass, sapphire, polymer, or any suitable material, as may be known to one of skill in the art. The display stack 109 may include a liquid crystal display (LCD), an organic light emitting diode display (OLED), or any suitable display technology. The display stack 109 outputs a graphical user interface of the device and may include other sensors, such as a fingerprint sensor, touch sensors, force sensors, and the like. The touch and/or force sensors may detect various types of user interactions including swipes, taps, and other gestures. Generally, the cover 108 defines a front surface of the device 100. In some cases, the cover 108 defines the entire front surface of the device 100. In other examples, the cover 108 and the housing 102 collectively define the front surface of the device 100. The housing 102 may be flush with respect to the cover and may entirely surround the cover 108.

The device 100 may also include a wristband 104 that secures the device 100 to a user's wrist. In some cases, the wristband 104 is detachably coupled to a recess or other feature of the housing 102. In some cases, the coupling portion of the wristband 104 may be formed from the same material as the housing 102. In other examples, the wristband 104 is formed from other materials to provide a visually-distinct appearance.

The housing 102 may define an external side surface 102a along which various user input controls may be positioned. For example, the device 100 includes a side button 110 and a crown 112 positioned along (and which may partially protrude from) the external side surface 102a. The button 110 may be configured to be translated inward in response to a user input, and may be programable such that the user can define a particular function that is controlled by the button.

The crown 112 may protrude from the external side surface 102a of the housing 102 and may be external to the housing 102 (e.g., the rotating or translating components of the crown do not extend to the internal volume of the device). More specifically, a cap of the crown 112 (and optionally other crown components) may be external with respect to a sidewall of the housing 102 which defines the internal volume. The cap of the crown 112 may define a generally round shape with texture, knurling, grooves, or other patterns that facilitate gripping of the crown assembly (e.g., during rotation of the crown). In some cases, the crown 112 is configured to receive a variety of inputs through which users can interact. For example, a user may rotate the crown 112 and/or may translate the crown 112 to zoom, scroll, rotate, select, or otherwise change the user interface of the display stack 109. In some cases, rotation of the crown 112 may be detected via a magnetic sensor, such as a tunneling magnetoresistance (TMR) sensor, hall effect sensor, or the like. In some cases, the rotation of the crown 112 may be detected by non-magnetic sensors, such as optical sensors.

In some examples, the crown 112 can receive biometric signals from a user. For example, the crown assembly may be coupled to biometric sensing circuitry or include or define conductive paths to biometric circuitry within the device 100. More particularly, the cap of the crown 112 may define a user input surface 112a through which biometric signals can be received. For example, the device 100 may receive heart rate and/or ECG signals via a user contacts the crown assembly 112 at the user input surface 112a. Specifically, the sensors may receive the signals when the user opens an application or otherwise activates the function, in some examples. More generally, the user input surface 112a may be a conductive surface (e.g., formed from a metal or similar materials as the housing) that is coupled to biometric sensors or other biometric sensing circuitry. When used to conductively couple to a user's skin for detecting signals (e.g., voltage signals), the user input surface 112a may be referred to as an electrode.

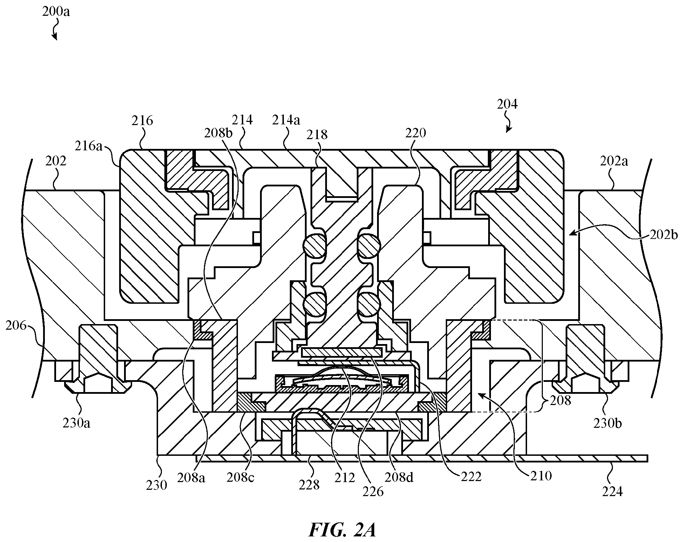

FIGs. 2A-2B show partial cross-sectional views of the crown along line 2A-2A of FIG. 1A. In these examples, the crown is positioned within a recess of the housing, yet the crown (e.g., the rotating and/or moving portions of the crown) remains external with respect to an inside volume of the device. The rotation of the crown is detected through a sidewall, also referred to as a wall segment of the housing. More particularly, the rotation of the crown is detected via a change in a magnetic field that is measured through a solid portion or bulk of the sidewall material (e.g., the change in a magnetic field may be detected as a change in resistance, voltage, or the like at the magnetic sensor). Thus, the crown can remain be external to an otherwise sealed or enclosed internal volume despite the internal positioning of the magnetic sensing system. In this configuration, the moving portions of the crown do not extend into the internal volume and thus coupling features that would be used to retain the crown within the internal volume of the device and which would be used to seal the internal volume from contaminants may be omitted (at least in the area of the crown).

As shown in FIG. 2A, a device 200a may include a housing 202 to which a crown 204 is coupled. The housing 202 may define an external surface 202a of the device 200a. As discussed above, the external surface 202a may be a side surface of the device. Generally, a portion of the crown 204 protrudes from the external surface 202a such that a user may interact with the crown 204 (e.g., rotate, press, and the like).

The housing 202 may define a recessed portion 202b. The recessed portion 202b (which defines a recess) may be configured to receive a portion of the crown 204. In some embodiments, the recessed portion 202b is defined by a main body 206 of the housing and a bucket assembly 208. The main body 206 may define a substantial portion of the housing 202 and may at least partially define the internal volume 210 of the device 200a. The bucket assembly 208 may be positioned within an opening defined by the main body 206. The recessed portion 202b may define a cavity along the housing 202 that is fluidly decoupled from the internal volume 210 of the device 200a. More particularly, the bucket assembly 208 may be positioned at an end of the opening of the main body 206 and may seal the internal volume 210 with respect to the recessed portion 202b. The bucket assembly 208, and the recessed portion 202b more generally, may define a blind hole along the exterior side of the housing 202.

The bucket assembly 208 may be coupled to the main body 206 via a coupling member 208a. The coupling member 208a may be an adhesive. In some embodiments, the coupling member 208a is an injection molded piece (e.g., overmolded) made from a thermoplastic polymer. In some examples, the bucket assembly 208 is positioned within the main body 206 and the coupling member 208a is then injection molded to couple the bucket assembly 208 to the main body 206 and create a seal and/or transition between both pieces. In some cases, the bucket assembly 208 may be coupled to the main body 206 (e.g., at the location indicated by the coupling member 208a) via welding, brazing, soldering, or another fusion bond or metal-to-metal coupling technique. The bucket assembly 208 may also include a retainer portion 208b which accepts a portion of the crown 204. In some cases, the bucket assembly 208 defines a wall segment 208d (also referred to as a sidewall) which may be coupled to the retainer portion 208b via coupling member 208c. In some examples, the coupling member 208c may be weld material, solder material, an adhesive, an injection molded (e.g., overmolded) piece, or the like. In some cases, the coupling member 208c electrically isolates the wall segment 208d from the retainer portion 208b. The wall segment 208d may be configured to provide structural support (e.g., in a direction of pressing) to the crown 204.

In some cases, a dome switch 212 may be coupled to the wall segment 208d. The dome switch 212 may be configured to bias the crown 204 to an unpressed position (e.g., in a direction away from the housing 202 and/or the internal volume 210) and collapse in response to a user press input having a threshold force. After releasing the press input, the dome switch 212 restores to an unactuated position, where the crown is biased away from the bottom surface of the recess. Actuating the crown may cause the dome switch 212 to produce a tactile feedback to the user. As the dome switch 212 is external to the interior volume of the device, the dome switch may provide haptic functionality but not input sensing functionality.

In some cases, the bucket assembly 208 may be a monolithic piece defining a sub-cavity that houses a portion of the crown 204 and the dome switch 212. In some configurations, the housing 202 defines a blind hole in which the crown is positioned. The blind hole may be defined by the main body 206 and the bucket assembly 208. In some examples, however, the blind hole may be formed into a unitary housing structure 202.

Generally, the crown 204 may include a cap 214, a knob portion 216, a shaft 218, and a collar 220. The cap 214 may define a first portion of a user input surface 214a of the crown 204, which generally protrudes from the external surface 202a. The first portion of the user input surface 214a is configured to receive a user input. The user input may include a pressing input, which actuates the crown 204 towards the internal volume 210 of the device 200a. In some cases, the user input may be a touch input.

The user input may additionally and/or alternatively be an electrocardiogram (ECG) signal. The ECG signal may be detected at the first portion of the user input surface 214a through contact with a user's skin. The ECG signal may be transmitted from the cap 214, to the shaft 218, and to a conductive member 222 conductively coupled to the shaft. In turn, the conductive member 222 may be conductively coupled to the wall segment 208d and to a circuit element 224 within the internal volume 210 of the device 200a. In some cases, the circuit element 224 may be a flexible circuit, a conductive via, or other electrically conductive element configured to convey a signal. The crown 204 may include a knob portion 216 which may be electrically isolated from the cap 214 and from the user input surface 214a.

The knob portion 216 of the crown 204 may be configured to receive a rotational input from the user via a second portion of the user input surface 216a. Upon receiving the rotational input, the knob portion 216, the cap 214, and the shaft 218 may be configured to collectively rotate. The rotation of the crown 204 is detected by one or more sensing systems of the device 200a. The rotational input may cause display of different user interface elements and/or allow the user to scroll or navigate the display of the device 200a. In some examples, the rotational input may be used as a volume control, a brightness control, or the like.

The one or more sensing systems of the device 200a may include a magnetic sensing system. In this example, the crown 204 may include a magnetic member 226 coupled to the shaft 218. As shown, the magnetic member 226 may be positioned at the end of the shaft 218 closest to the dome switch 212 and closest to the wall segment 208d. The magnetic member 226 may include opposite polarities which generate a change in magnetic field when the crown 204 rotates. For example, the magnetic member 226 may include a north polarity portion and a south polarity portion diametrically distributed with respect to the shaft 218. It should be understood that the magnetic member 226 may refer to more than one magnetic piece distributed along the sidewall (e.g., multiple pieces along the periphery of the shaft 218).

In this example, the crown 204 and the dome switch 212 are positioned externally with respect to the internal volume 210 at a first side of the wall segment 208d. The first side of the wall segment 208d may be referred to as an external side. As depicted, a magnetic sensing system 228 is positioned within the internal volume 210 at a position opposite the dome switch 212 and the shaft 218. That is, the magnetic sensing system 228 may be positioned on a second side of the wall segment 208d. The second side of the wall segment 208d may be referred to as an internal side. In this configuration, the magnetic sensing system 228 detects the change in the magnetic field due to the rotation of the magnetic member 226 through the wall segment 208d. Due to this configuration, the crown 204 (including rotating members of the crown 204) may be external with respect to the internal volume, thereby improving the integrity of the environmental sealing of the internal volume of the device (e.g., leak paths are reduced and/or completely eliminated). In addition, due to its external structure, less sealing members and/or coupling members in the crown further reduces the complexity of the crown. In some cases, the size of the crown 204 may be decreased.

Generally, the magnetic sensing system 228 may include a magnetic sensor. As the magnetic member 226 rotates, the magnetic field that is propagated by the rotating magnets (and which is ultimately detected at the magnetic sensor) moves and/or changes, and thereby a rotation and rotational speed of the magnetic member 226 (and thus, of the crown 204) can be detected. In some examples, the magnetic sensor is a tunnelling magnetoresistance (TMR) effect sensor. As the magnetic member rotates, the resistance of the magnetic sensor changes as a function of the angle between the magnetization directions of a free layer and a pinner layer. More generally, the TMR sensor detects the changes in the magnetic field that are produced when the magnets coupled to the crown 204 rotate. In other examples, the magnetic sensor is a hall effect sensor, an anisotropic magnetoresistance (AMR) effect sensor, a giant magnetoresistance (GMR) effect sensor, or the like.

Regardless of the type of magnetic sensor, in some embodiments, one or more (and optionally two or more) magnetic sensors are positioned at different orientations. For example, a first magnetic sensor may be at a first orientation with respect to the sidewall and the second magnetic sensor may be at a second orientation with respect to the sidewall, where the first and second orientations are different. More particularly, the first and second magnetic sensors may be oriented differently such that the detected changes in the magnetic field from the rotating crown are approximately 90 degrees out of phase. For example, both magnetic sensors may be coupled to a common surface that is parallel to the wall segment and positioned orthogonally with respect to each other. The different orientations of the magnetic sensors relative to each other enables a processing system within the device 200a to detect a direction of rotation, such as clockwise and counterclockwise, of the magnetic member 226 and, thus, of the crown 204. More generally, the magnetic sensors may be oriented relative to one another such that a quadrature encoded signal may be produced, thereby facilitating detecting a rotation direction in addition to a rotation speed (and optionally a rotational position). In some cases, the magnetic sensors are positioned approximately ninety degrees with respect to each other, though other angles are envisioned. The output from the two or more magnetic sensors may be transmitted to a processing system of the device 200a via the circuit element 224.

In some embodiments, the magnetic sensing system 228 may be coupled to the housing 202. For example, the magnetic sensing system 228 may be mounted on a bracket 230 and secured to the housing 202 via fasteners 230a and b. In this example, an air gap may be defined between the magnetic sensing system and the wall segment 208d. In some configurations, the magnetic sensing system 228 may be coupled to the wall segment 208d. For example, the circuit element 224 may be coupled to the wall segment 208d and the magnetic sensing system 228 may be coupled to the circuit element 224 (e.g., the circuit element may be between the wall segment 208d and the magnetic sensing system 228).

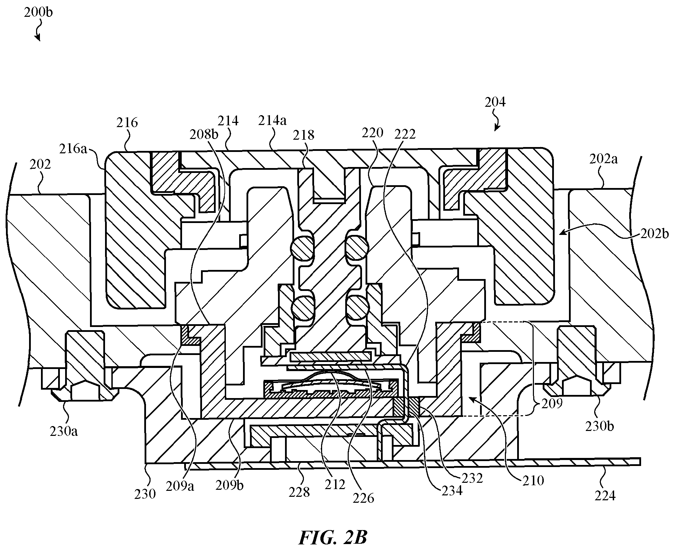

FIG. 2B shows a cross-sectional view of device 200b, which is a variation of device 200a from FIG. 2A. In this view, a bucket assembly 209 is formed as a single structure with a via or through-conductor to facilitate conductive coupling to the crown (e.g., for passing ECG signals). In particular, the bucket assembly 209 may include a sidewall 209b which defines a side portion of the recess or recessed portion 202b as well as an end portion of the recess or recessed portion 202b (e.g., a bottom of the recess). The sidewall 209b may also define a ledge that is configured to couple to a complementary ledge on the housing 202 (and may be secured via a coupling member 209a, which may correspond to coupling member 208a from FIG. 2A). The bucket assembly 209 may also be welded, brazed, soldered, etc., to the housing 202. Because the sidewall 209b is built as a unitary member, the bucket assembly 209 provides more structural support to the dome switch 212 and to the crown 204.

At the end portion of the recess, the sidewall 209b may define an opening. This opening is filled with an insulation member 232 and a via 234 that is conductively isolated from the sidewall 209b. In some cases, the insulation member 232 may be formed using an overmolding process and may be formed from a polymer, plastic, or other electrical insulator. The via 234 may be central with respect to the opening and the insulation member 232 may surround the via 234. The via 234 may be a piece of conductive material such as a metal, a circuit element (e.g., a printed circuit board or flexible circuit element), or any other suitable conductive structure. The conductive member 222 is conductively coupled to the via 234. The via 234 is also coupled to the circuit element through one or more intermediate members.

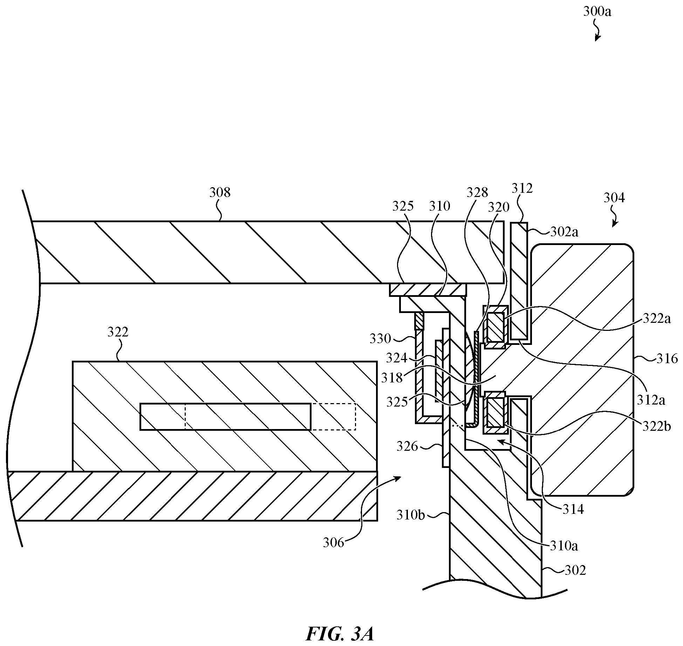

As described with respect to FIGs. 2A and 2B above, the crown 204 is external with respect to an internal volume 210 of the device 200a and b. This positioning of the housing 202 is achieved by having a recessed portion 202b in the housing 202 in which the external crown 204 is inserted. FIGs. 3A-4B depict different configurations of an external crowns having different housing configurations that also enable the crown to be positioned externally with respect to the internal volume (or "dry" or "sealed" volume) of the device.

FIG. 3A depicts a cross sectional view of a device 300a having an external crown 304. In this example, a housing 302 of the device 300a defines one or more features configured to retain the crown externally with respect to an internal volume 306. Due to the geometry and/or structure of the fully external crown with respect to the internal volume, the sealing quality of the internal volume may be improved. In other words, the crown structure reduces possible leak paths and thus the internal volume is better protected from water or contaminant ingress. In some cases, the external structure of crown may further reduce the complexity and size of the crown as less or no seals and/or components are used to prevent water ingress into the internal volume. As a result, a size of a display assembly 308 may be increased due to a reduced overall footprint of the crown 304.

In some examples, the housing 302 comprises a first sidewall 310 and a second sidewall 312. The first sidewall 310 may define the internal volume 306 of the device 300a in which electronic components of the device 300a are housed. In some cases, the first sidewall 310 may be a unitary member of the housing 302 and may be configured to prevent ingress of liquid to the internal volume 306. In addition, the first sidewall 310 may be configured as a structural support for one or more electronic components or mechanical members of the device 300a. For example, the first sidewall 310 may be configured to at least partially support the display 308. In some cases, the first sidewall 310 may be configured to couple to one or more internal platforms within the device.

The housing 302 may further comprise a second sidewall 312. The second sidewall 312 may define a portion of an external surface of the housing 302a. Generally, the second sidewall 312 may be formed as a unitary member of the housing 302. The second sidewall 312 may extend substantially parallel with respect to the first sidewall 310. In some cases, the first sidewall 310 and the second sidewall 312 define a secondary volume 314 that is captured between both sidewalls. Within the secondary volume 314, crown components, switches, or other external electronic components may be housed yet remain occluded from the outside. In some embodiments, the secondary volume 314 is fluidly coupled to an external environment. Further, the secondary volume 314 may be fluidly decoupled from the internal volume 306 (e.g., not part of the sealed internal volume of the device). Accordingly, the components housed within the secondary volume 314 are external to the internal volume 306 and may be configured for external conditions, such as water ingress.

As depicted in the figure, the device 300a may include a crown 304 coupled to the second sidewall 312. The crown 304 may have similar or the same components as crown 204 from FIGs. 2A and 2B. In particular, the crown 304 may include a cap 316 configured to receive a user input (e.g., a pressing or actuation input, or a rotational input). The crown 304 may also include a shaft 318 that extends from the cap 316 and which is configured to transmit the pressing and/or rotational input from the user interface surface to one or more actuators or sensors. In the configuration shown, the second sidewall 312 defines an opening 312a through which the shaft 318 extends. In some cases, the shaft 318 extends from the cap 316, through the opening 312a, and into the secondary volume 314.

In some examples, the crown 304 may also include a magnetic assembly 320. The magnetic assembly may be positioned within the secondary volume 314 and may be coupled to the shaft 318. In some cases, the magnetic assembly 320 may be configured to retain the crown 304 with respect to the device 300a. For example, the magnetic assembly 320 may be configured to fix the crown 304 in a horizontal direction with respect to the page. The magnetic assembly 320 may further be configured to rotate with the crown 304 in response to the rotational input. Further, the magnetic assembly 320 may be configured to shift with the crown in response to the pressing input. In some cases, the magnetic assembly 320 includes a C-clip or other retention member. Generally, the magnetic assembly 320 may have a larger diameter than the diameter of the shaft 318 to securely retain the crown 304 within the second volume. In some cases, the magnetic assembly 320 is installed after installing the crown 304 to fix the crown 304 with respect to the housing 302 and/or with respect to the second sidewall 312.

The magnetic assembly 320 may include at least one magnetic member, such as magnetic member 322a and 322b. As discussed in FIGs. 2A and 2B above, the magnetic member may be formed from or include permanent magnets. In some cases, magnetic member 322a has an opposite polarity from magnetic member 322a. As the crown 304 rotates, the magnetic assembly 320 rotates with the shaft 318, and thus the magnetic members 322a and 322b rotate. The rotation of the magnetic members 322a and 322b generate a change in a magnetic field that may be detected by a magnetic sensing system 324 positioned within the internal volume 306, which may include one or more magnetic sensors as described as to magnetic sensing system 228 above.

More generally, the magnetic assembly 320, along with magnetic member 322a and 322b, may be positioned on or adjacent to a first side 310a of the first sidewall 310. The first side 310a may be referred to as an external side due to its exposure to an external environment. The magnetic sensing system 324 may be positioned on or adjacent to a second side 310b of the first sidewall 310, opposite the first side 310a. Accordingly, the magnetic sensing system 324 detects a change in the magnetic field due to rotation and/or due to actuation of the crown through the first sidewall 310. In some embodiments, a circuit element 326 may be conductively coupled to the second side 310b. The magnetic sensing system 324 may be coupled to the circuit element 326. In other examples, the magnetic sensing system 324 may define a gap with respect to the first sidewall 310, such as with the structure described in FIG. 2A.

In some examples, the device 200a includes a dome switch 325. The dome switch 325 may be positioned within the secondary volume 314. Specifically, the dome switch 325 may be positioned to the first side 310a and may be coupled to the first sidewall 310. The dome switch 325 may be configured to bias the crown 304 in a direction away from the first sidewall 310 and collapse in response to a threshold force applied to the crown 304 to produce a haptic feedback. In some cases, the dome switch 325 may provide a haptic or tactile response in response to actuation of the crown. As the dome switch 325 is external to the interior volume of the device, the dome switch may provide haptic functionality but not input sensing functionality. The magnetic sensing system 324 may be configured to detect the translation or shift of the crown. In some examples, the dome switch 325 may be an electro-mechanical switch conductively coupled to circuit element 326 and configured to generate an output in response to an actuation of the switch. Regardless of the type of dome switch 325, the dome switch 325 may be open to an external environment via the secondary volume 314.

In some cases, the device 200a may additionally include a conductive member 328. The conductive member 328 may configured to convey a signal from the user input surface of the crown 304 to other circuitry within the device to facilitate further input processing. For example, the crown 304 may be configured to receive an ECG signal at the user input surface of the cap 316. The ECG signal may be transmitted through the shaft 318 and to the conductive member 328. The conductive member 328 may be conductively coupled to the circuit element 326 through the first sidewall 310 (e.g., where the first sidewall is formed from a metal material). In some embodiments, the first sidewall 310 may include one or more vias that couple to the conductive member 328 and which are isolated from the sidewall due to an insulating member, such as described in FIG. 2B. While vias and/or sealed members for ECG signals are shown, these vias and/or sealed members are optional. In some examples, the vias are omitted and the first sidewall 310 does not have additional recesses and/or pass-through connections from the crown and other haptic feedback members.

In some cases, the device may include magnetic shielding members. Generally, other electronic components within the device 200a and/or other accessories, such as charging accessories, may generate changes in a magnetic field. Due to the position and/or size of other components, these changes in the magnetic field may be detected by the magnetic sensor and/or magnetic sensing system 324. For example, the device 300a may include a haptic engine 332. The haptic engine 332 can be configured to generate a vibration or haptic output. In some cases, the haptic engine 332 includes one or more magnetic members that are actuated to produce a vibration. Similarly, the device 200a may be configured to interoperate with a magnetic wireless charger. In response to the device 200a being connected to a magnetic wireless charger, a magnetic field may be generated to charge a battery the device 200a. To mitigate the effects of magnetic fields from other sources (e.g., chargers, haptic engines), in some cases, a magnetic shield 330 may be positioned between the magnetic sensing system 324 and one or more electronic components, such as the haptic engine 332 to shield the magnetic sensing system 324 from magnetic fields from sources other than the magnetic assembly 320. Generally, the magnetic shield 330 may at least partially surround the magnetic sensing system 324. In some cases, the magnetic shield 330 may be formed from a ferromagnetic material, such as iron, nickel, or cobalt and/or materials with a high relative permeability.

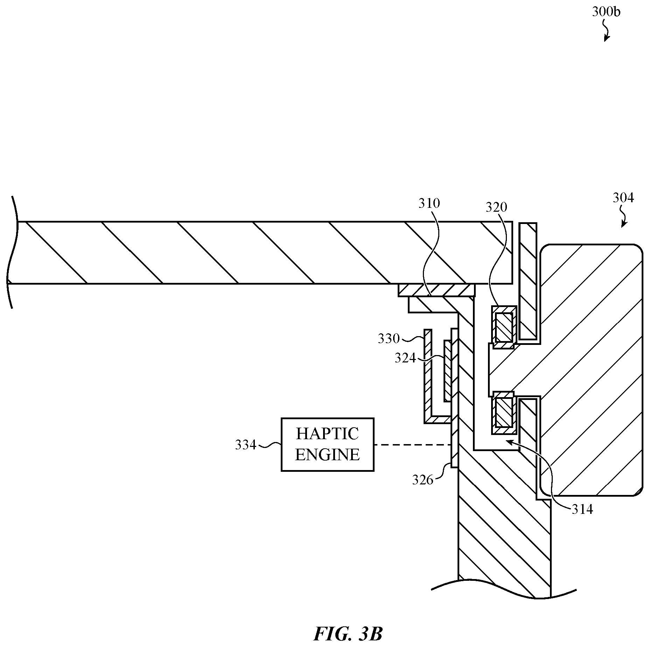

FIG. 3B shows a cross-sectional view of an electronic device 300b, which may be a variation of electronic device 300a. In this variation, the electronic device 300b does not include a mechanical actuation switch which may produce a haptic response, such as a dome switch. In this configuration, a haptic engine 334 may be configured to produce a haptic output to signify when an input has been detected or registered, or for other haptic or tactile outputs at the crown. In response to detecting an input from the crown 304, such as an actuation or a rotational input, the haptic engine 334 may produce a generalized haptic response. In some cases, the haptic engine 334 may be a localized haptic engine configured to generate a local haptic response at the crown 304. In this configuration, fewer components and/or electrical connections are exposed to an external environment.

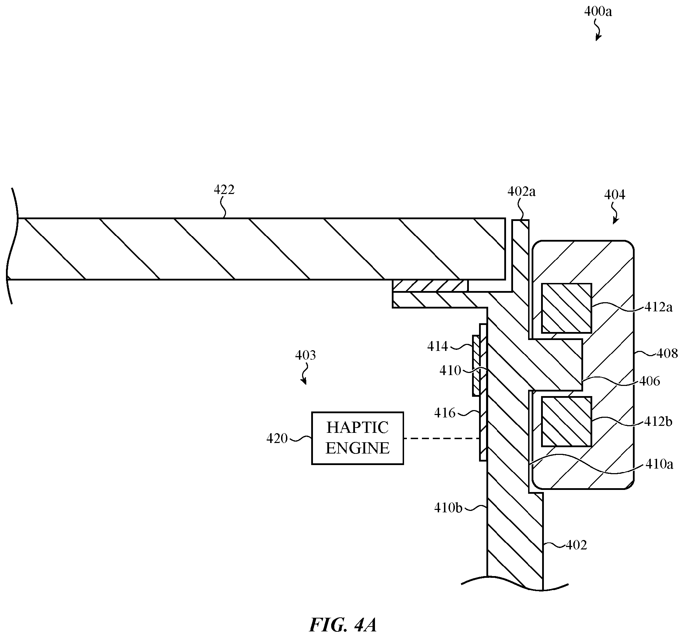

In some embodiments, the housing of a device may define one or more features, such as a protrusion configured to couple to or support an external crown. In these embodiments, the crown is completely external with respect to an external surface of the device, which may further reduce the amount of internal space that is dedicated to a crown and/or its systems. FIG. 4A depicts a cross-sectional view of a device 400a having a protruding member defined by the housing of the device. Unlike the examples depicted in FIGs. 2A-3B above, the crown does not include a shaft. Instead, the housing defines a protruding member which couples to the crown and provides a rotational-bearing surface to the crown. The protruding member is static.

The device 400a may include a housing 402 which defines an external side surface 402a of the device. In some cases, a display 422 may be coupled to the housing 402 and define, along with a portion of the housing 402, a flush front surface of the device 400a. Additionally, the housing 402 may define an internal volume 403 of the device 400b that may be fluidly isolated from the external environment. In some examples, the housing 402 may further define an external protrusion 406 which is proud with respect to the external side surface 402a and which is configured to couple to the crown. In some examples, the housing 402 of the device 400a may be a monolithic housing.

A crown 404 may be configured to be rotatably coupled to the external protrusion 406. Specifically, the crown 404 may include a cap 408 that defines a user interface surface that is configured to receive a rotational input. The cap 408 may be a monolithic piece that collectively defines a knob portion and an end of the crown 404. The crown 404 may include one or more rotational bearings that couple to the external protrusion 406, allowing the cap 408 to rotate, while retaining the crown 404 in place.

In some cases, the crown 404 may include one or more magnetic members 412a and 412b. The one or more magnetic members 412a and 412b may be positioned peripherally with respect to the external protrusion 406 and are configured to rotate with the crown 404 in response to a rotational input. In some cases, as depicted, the one or more magnetic members 412a and 412b may be embedded within a portion of the crown 404. In some examples, the one or more magnetic members 412a and 412b may be coupled to a surface of the crown 404 that is opposite to the user interface surface (e.g., the innermost surface of the crown). In this configuration, the magnetic members 412a and 412b are adjacent to a first side 410a of a sidewall 410 of the housing 402.

The device 400a may include a magnetic sensing system 414 positioned on a second side 410b of the sidewall 410, opposite the first side 410a. Accordingly, as the one or more magnetic members 412a and 412b rotate, the magnetic sensing system 414 is configured to detect a change is magnetic field due to the rotation through the wall of the housing. The magnetic sensing system 414 may be coupled to a circuit element 416. The circuit element 416 may be coupled to the second side 410b of the sidewall 410.

In some cases, the cap 408 of the crown 404 may be conductively coupled to the external protrusion 406. Due to the conductive coupling, the cap 408 may be configured to receive an ECG signal from the user's skin and convey the signal via the external protrusion 406 and to the circuit element 416. The ECG signal may be detected by the circuit element 416 and thus a processor unit within the device. Due to the static configuration of the external protrusion 406, conductive members that transmit signals while isolating a shear force may be reduced and/or eliminated.

While in the configurations of FIGs. 2A-3B, the crown could be configured to be pressed and/or actuated with respect to the housing, in the example shown in FIG. 4A, the crown may be translatably fixed with respect to the housing (e.g., may not move when pressed). In this configuration, the crown 404 may include a touch sensor at the user input surface. The touch sensor may be configured to detect a touch input and may be communicatively coupled to the circuit element 416. In response to the touch input, an output from a haptic engine 420 may be triggered. The output may from haptic engine 420 may be felt by the user at the crown 404 and may resemble the sensation of pressing or collapsing a button with a dome switch.

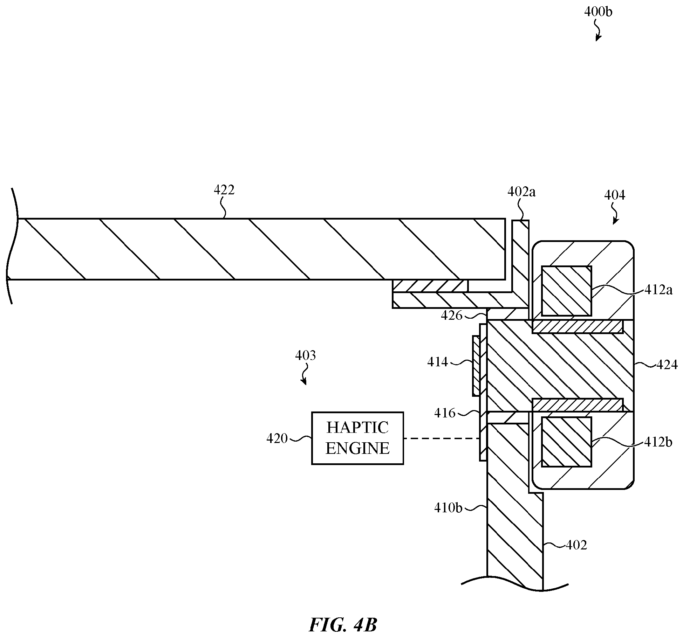

FIG. 4B shows a cross sectional view of a device 400b, which may be a variation of device 400a from FIG. 4A. In this variation, the housing 402 includes an external protrusion 424 which is coupled to the housing 402. The external protrusion 424 is electrically isolated from the housing 402 by an insulating member 426. The insulating member 426 may be formed from a polymer, glass-ceramic, composite, or other type of electrically insulating material. In some cases, the insulating member 426 may be overmolded. The housing 402 and/or the external protrusion 424 may define one or more openings, recesses, or features for securely coupling to the insulating member 426. In this case, the circuit element 416 is coupled to an internal surface of the external protrusion 424. Thus, signals received at a user input surface of the crown 404 are conveyed directly through the external protrusion 424 (e.g., the ECG signal). As explained above, the magnetic members 412a and 412b (positioned externally to the housing 402) may produce a change in a magnetic field due to rotation which can be detected by a magnetic sensing system 414 (positioned within the internal volume 403) through the sidewall 410 and/or through a portion of the external protrusion 424.

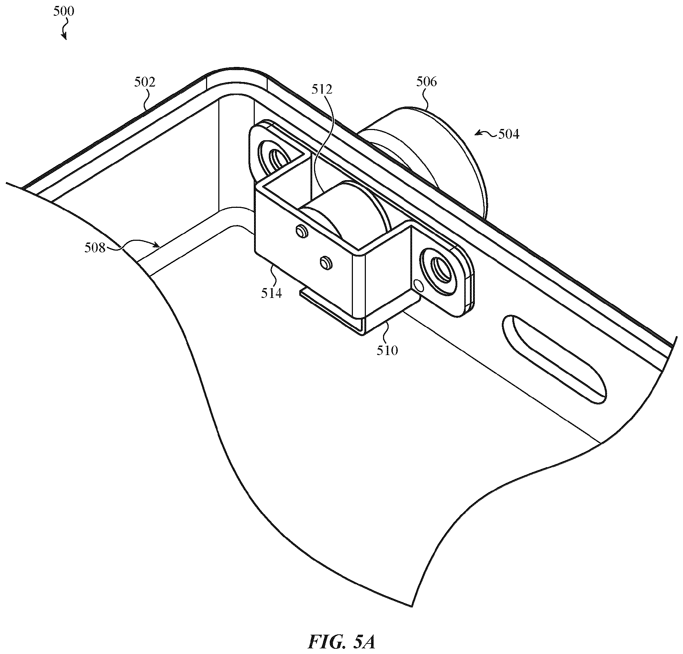

FIGs. 5A-5D shows an example electronic device 500 with a crown 504 that includes a magnetic sensor for detecting rotation. In this example, the magnetic sensing system (including a magnetic member of a rotating structure of the crown) is completely within the internal volume 508 of the device 500.

The device 500 may include a housing 502, which may be formed from a metal, and define the internal volume of the device 500. In some cases, the housing may include one or more openings through which user input members, such as the crown 504 and other buttons, extend. In this case, the crown 504 may extend through a portion of the housing to securely retain it to the device 500. In other cases, the crown 504 may be completely external to the housing 502, such as in examples described above. The crown 504 may include a cap 506 defining a user input surface which allows a user to rotate the crown 504 and/or provide a touch input to the device. In some cases, the cap 506 may include knurling or other textured features. Within the internal volume 508, the device 500 may include a mounting bracket 510. The mounting bracket is configured to securely couple the crown 504 and/or a magnetic sensing assembly 512 to the housing.

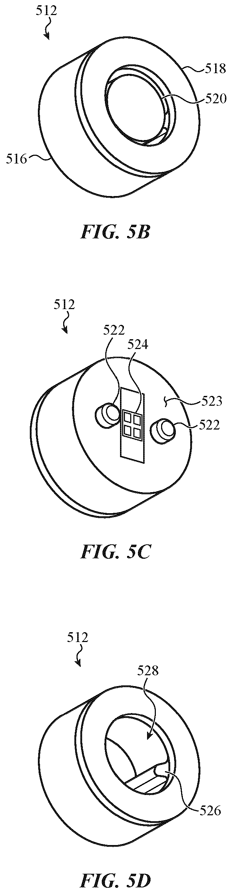

As shown in FIG. 5B, the magnetic sensing assembly 512 may include a sub-enclosure 516, a sealing member 518, and a magnetic member 520. The magnetic member 520 may be coupled to an end of a crown shaft such that the magnetic member 520 is configured to rotate in response to a rotational input of the crown 504. In this example, the crown shaft may extend through the housing to couple to the magnetic member 520. In some cases, the magnetic member 520 may be the shaft of the crown 504 and extend at least partially into the cap 506. In some embodiments, the crown 504 may be magnetically coupled to the magnetic member 520 while being fully external to the housing (e.g., the magnetic member 520 and the crown 504 may not be mechanically coupled). The magnetic coupling of the crown 504 to the magnetic member 520 may be configured to cause rotation of the magnetic member 520 in response to a rotation of the crown 504.

In some cases, the sealing member 518 is coupled to an open end of the sub-enclosure 516. When mounted to the housing, the sealing member 518 deforms against the housing, thereby creating a seal between the housing 502 and/or the bracket 510. The sealing member 518 may be formed from a compliant material, such as rubber, silicone, vinyl, neoprene, nitrile, and the like.

As shown in FIG. 5C, the magnetic sensing assembly 512 includes a magnetic sensor 524 positioned over the closed end of the sub-enclosure 516. In this example, the magnetic sensor 524 is positioned on a first side 523 that is external with respect to an internal cavity 528 (FIG. 5D) of the sub-enclosure 516. The magnetic member 520 is positioned on a side opposite to the first side 523 and within that internal cavity 528.

Generally, the sub-enclosure 516 is configured to house the magnetic member 520. The magnetic member 520 may be friction fit within the sub-enclosure 516 while being free to rotate. As the magnetic member 520 rotates in respect to a user rotational input, the magnetic sensor 524 is configured to detect a change in magnetic field due to the rotation of the magnetic member 520 through a wall of the sub-enclosure 516.

As shown in FIG. 5D, the sub-enclosure 516 may include a plurality of ribs 526. The plurality of ribs 526 are configured to center the magnetic member 520 within the sub-enclosure 516. The plurality of ribs 526 may provide a friction fit to the magnetic member 520. In some cases, the magnetic member 520 may rotate against the plurality of ribs 526. Generally, the plurality of ribs 526 may include a coating to reduce friction between the plurality of ribs 526 and the magnetic member 520. The plurality of ribs 526 may be configured to deform against a surface of the magnetic member 520 to provide a secure yet rotatable coupling with respect to the sub-enclosure 516. In some embodiments, the plurality of ribs 526 and the sub-enclosure 516 are formed from a unitary piece.

Back to FIG. 5C, the sub-enclosure 516 may include a set of pins 522. In some cases, the set of pins 522 are configured to prevent rotation of the sub-enclosure 516 with respect to the housing. The set of pins 522 may be coupled to a magnetic shielding member 514 shown in FIG. 5A. The magnetic shielding member 514 may partially surround the magnetic sensing assembly 512 such to shield the magnetic member from detecting changes in a magnetic field due to other electronic components (e.g., haptic engine, wireless charging). In other words, the shielding member 514 reduces possible interferences with the detected change in magnetic field due to the rotation of the magnetic member 520.

These foregoing embodiments depicted in FIGs. 2A-5D and the various alternatives thereof and variations thereto are presented, generally, for purposes of explanation, and to facilitate an understanding of various configurations and constructions of a system, such as described herein. However, it will be apparent to one skilled in the art that some of the specific details presented herein may not be required in order to practice a particular described embodiment, or an equivalent thereof.

Thus, it is understood that the foregoing and following descriptions of specific embodiments are presented for the limited purposes of illustration and description. These descriptions are not targeted to be exhaustive or to limit the disclosure to the precise forms recited herein. To the contrary, it will be apparent to one of ordinary skill in the art that many modifications and variations are possible in view of the above teachings.

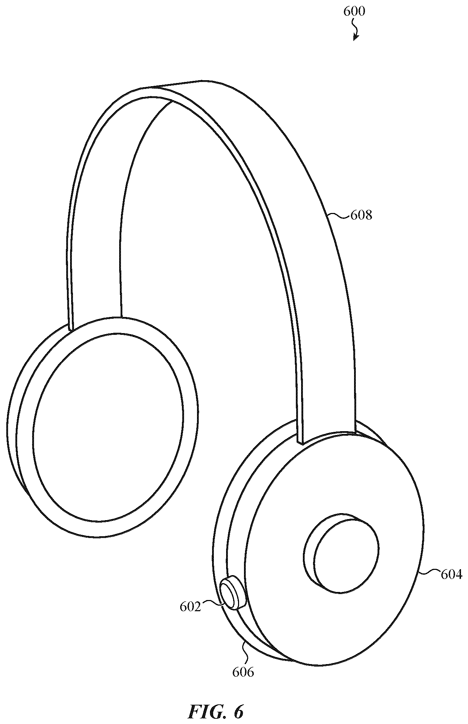

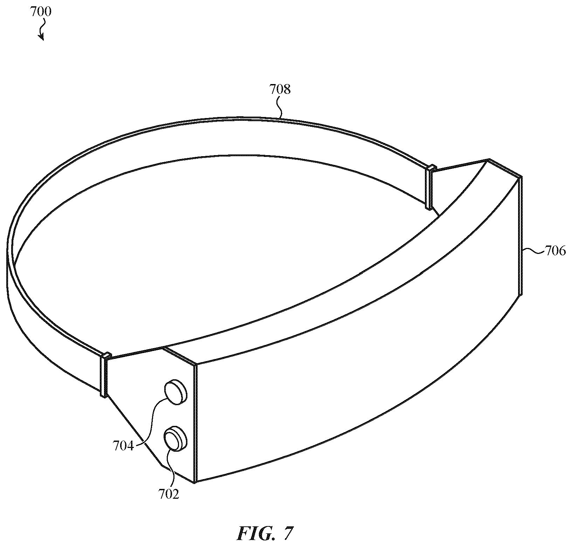

FIGs. 6 and 7 depict example electronic devices that may incorporate a crown, such as described in FIGs. 2A-5D. FIG. 6 depicts a wearable audio device and, in particular, headphones 600, which may incorporate a crown or similar input device. The headphones 600 may include a respective enclosure 604 configured to be worn over or within each ear of the wearer. In some embodiments, each enclosure may be coupled together via a headband 608 that fits over a user's head and which may balance a weight of each enclosure against the user's head. Generally, each enclosure 604 includes an audio output element 606 that is configured to output audio to each ear.

At least one enclosure 604 includes a crown 602, such as described in FIGs. 2A-5D. The crown 602 may be positioned along a side surface of the enclosure 604. In some cases, the crown 602 may be positioned over a surface opposite an audio output surface. The crown 602 may be configured to be accessed by the user in the worn position of the headphones 600. In some cases, the crown 602 may be used as a volume control, audio settings (e.g., next, pause, noise cancellation, hear-through) control, a call control, a smart-assistant control, power control, or the like.

FIG. 7 depicts a virtual reality device 700 that incorporates a crown or similar input devices described in FIGs. 2A-5D above. In some cases, the virtual reality device 700 includes an enclosure 706 and a band 708 configured to secure the enclosure 706 to a user's face. The virtual reality device 700 includes a crown 702 (see, e.g., crown described in FIGs. 2A-5D) and other input devices 704. The enclosure 706 may include one or more displays and/or audio output elements. In various embodiments, a graphical output of the display(s) is responsive to inputs provided to the crown 702 and/or other input devices 704. In various embodiments, an audio output of the audio output element(s) may be provided within the enclosure 706. In some cases, the crown 702 may be used as a zoom in/out control, focus control, display control, volume control, audio settings (e.g., next, pause, noise cancellation, hear-through) control, a call control, a smart-assistant control, power control, or the like.

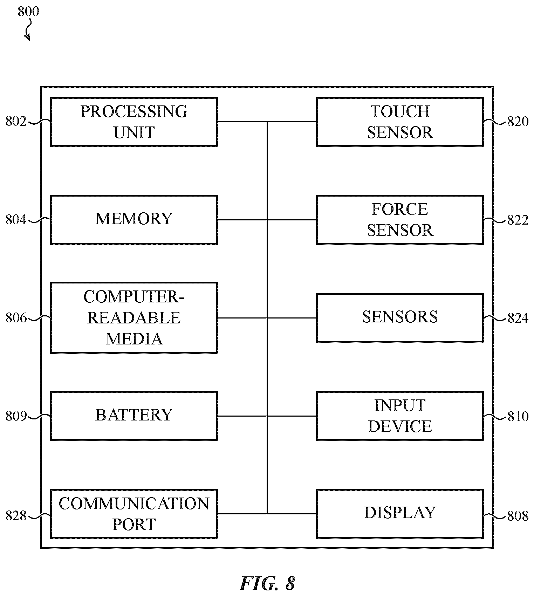

FIG. 8 depicts an example schematic diagram of an electronic device 800. The device 800 of FIG. 8 may correspond to the wearable electronic device 100 shown in FIG. 1A, device 600 of FIG. 6, device 700 of FIG. 7, or other electronic devices. To the extent that multiple functionalities, operations, and structures are disclosed as being part of, incorporated into, or performed by the device 800, it should be understood that various examples may omit any or all such described functionalities, operations, and structures. Thus, different examples of the device 800 may have some, none, or all of the various capabilities, apparatuses, physical features, modes, and operating parameters discussed herein.

As shown in FIG. 8, a device 800 includes a processing unit 802 operatively connected to computer memory 804 and/or computer-readable media 806. The processing unit 802 may be operatively connected to the memory 804 and computer-readable media 806 components via an electronic bus or bridge. The processing unit 802 may include one or more computer processors or microcontrollers that are configured to perform operations in response to computer-readable instructions. The processing unit 802 may include the central processing unit (CPU) of the device. Additionally or alternatively, the processing unit 802 may include other processors within the device including application specific integrated chips (ASIC) and other microcontroller devices.

The memory 804 may include a variety of types of non-transitory computer-readable storage media, including, for example, read access memory (RAM), read-only memory (ROM), erasable programmable memory (e.g., EPROM and EEPROM), or flash memory. The memory 804 is configured to store computer-readable instructions, sensor values, and other persistent software elements. Computer-readable media 806 also includes a variety of types of non-transitory computer-readable storage media including, for example, a hard-drive storage device, a solid-state storage device, a portable magnetic storage device, or other similar device. The computer-readable media 806 may also be configured to store computer-readable instructions, sensor values, and other persistent software elements.

In this example, the processing unit 802 is operable to read computer-readable instructions stored on the memory 804 and/or computer-readable media 806. The computer-readable instructions may adapt the processing unit 802 to perform the operations or functions described herein. In particular, the processing unit 802, the memory 804, and/or the computer-readable media 806 may be configured to cooperate with a sensor 824 (e.g., a rotation sensor that senses rotation of a crown component) to control the operation of a device in response to an input applied to a crown of a device (e.g., the crown 112 or any other crown described herein). The computer-readable instructions may be provided as a computer-program product, software application, or the like.

As shown in FIG. 8, the device 800 also includes a display 808. The display 808 may include a liquid-crystal display (LCD), organic light emitting diode (OLED) display, light emitting diode (LED) display, or the like. If the display 808 is an LCD, the display 808 may also include a backlight component that can be controlled to provide variable levels of display brightness. If the display 808 is an OLED or LED type display, the brightness of the display 808 may be controlled by modifying the electrical signals that are provided to display elements. The display 808 may correspond to any of the displays shown or described herein.

The device 800 may also include a battery 809 that is configured to provide electrical power to the components of the device 800. The battery 809 may include one or more power storage cells that are linked together to provide an internal supply of electrical power. The battery 809 may be operatively coupled to power management circuitry that is configured to provide appropriate voltage and power levels for individual components or groups of components within the device 800. The battery 809, via power management circuitry, may be configured to receive power from an external source, such as an AC power outlet. The battery 809 may store received power so that the device 800 may operate without connection to an external power source for an extended period of time, which may range from several hours to several days.

In some examples, the device 800 includes one or more input devices 810. An input device 810 is a device that is configured to receive user input. The one or more input devices 810 may include, for example, a crown input system (e.g., any of the crowns described herein), a push button, a touch-activated button, a keyboard, a keypad, or the like (including any combination of these or other components). In some examples, the input device 810 may provide a dedicated or primary function, including, for example, a power button, volume buttons, home buttons, scroll wheels, and camera buttons.

The device 800 may also include one or more sensors 824. The sensors 824 may detect inputs provided by a user to a crown of the device (e.g., the crown 112 or any other crown described herein). The sensors 824 may include sensing circuitry and other sensing components that facilitate sensing of rotational motion of a crown, as well as sensing circuitry and other sensing components (optionally including a switch) that facilitate sensing of translational and/or axial motion of the crown (or axial force applied to the crown). The sensors 824 may include components such as an optical sensing unit, a tactile or dome switch, or any other suitable components or sensors that may be used to provide the sensing functions described herein. The sensors 824 may also include a biometric sensor, such as a heart rate sensor, electrocardiograph sensor, temperature sensor, or any other sensor that conductively couples to the user and/or to the external environment through a crown input system, as described herein. In cases where the sensors 824 include a biometric sensor, it may include biometric sensing circuitry, as well as portions of a crown that conductively couple a user's body to the biometric sensing circuitry. Biometric sensing circuitry may include components such as processors, capacitors, inductors, transistors, analog-to-digital converters, or the like.

The device 800 may also include a touch sensor 820 that is configured to determine a location of a touch on a touch-sensitive surface of the device 800 (e.g., an input surface defined by the cover 108). The touch sensor 820 may use or include capacitive sensors, resistive sensors, surface acoustic wave sensors, piezoelectric sensors, strain gauges, or the like. In some cases, the touch sensor 820 associated with a touch-sensitive surface of the device 800 may include a capacitive array of electrodes or nodes that operate in accordance with a mutual-capacitance or self-capacitance scheme. The touch sensor 820 may be integrated with one or more layers of a display stack 109 to provide the touch-sensing functionality of a touchscreen. Moreover, the touch sensor 820, or a portion thereof, may be used to sense motion of a user's finger as it slides along a surface of a crown, as described herein.

The device 800 may also include a force sensor 822 that is configured to receive and/or detect force inputs applied to a user input surface of the device 800 (e.g., the cover 108). The force sensor 822 may use or include capacitive sensors, resistive sensors, surface acoustic wave sensors, piezoelectric sensors, strain gauges, or the like. In some cases, the force sensor 822 may include or be coupled to capacitive sensing elements that facilitate the detection of changes in relative positions of the components of the force sensor (e.g., deflections caused by a force input). The force sensor 822 may be integrated with one or more layers of a display stack 109 to provide force-sensing functionality of a touchscreen.

The device 800 may also include a communication port 828 that is configured to transmit and/or receive signals or electrical communication from an external or separate device. The communication port 828 may be configured to couple to an external device via a cable, adaptor, or other type of electrical connector. In some examples, the communication port 828 may be used to couple the device 800 to an accessory, including a dock or case, a stylus or other input device, smart cover, smart stand, keyboard, or other device configured to send and/or receive electrical signals.

The present disclosure recognizes that personal information data, including biometric data, in the present technology, can be used to the benefit of users. For example, the use of biometric authentication data can be used for convenient access to device features without the use of passwords. In other examples, user biometric data is collected for providing users with feedback about their health or fitness levels. Further, other uses for personal information data, including biometric data, that benefit the user are also contemplated by the present disclosure.

The present disclosure further contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure, including the use of data encryption and security methods that meets or exceeds industry or government standards. For example, personal information from users should be collected for legitimate and reasonable uses of the entity and not shared or sold outside of those legitimate uses. Further, such collection should occur only after receiving the informed consent of the users. Additionally, such entities would take any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices.

Despite the foregoing, the present disclosure also contemplates embodiments in which users selectively block the use of, or access to, personal information data, including biometric data. That is, the present disclosure contemplates that hardware and/or software elements can be provided to prevent or block access to such personal information data. For example, in the case of biometric authentication methods, the present technology can be configured to allow users to optionally bypass biometric authentication steps by providing secure information such as passwords, personal identification numbers (PINS), touch gestures, or other authentication methods, alone or in combination, known to those of skill in the art. In another example, users can select to remove, disable, or restrict access to certain health-related applications collecting users' personal health or fitness data.

As used herein, the phrase "at least one of" preceding a series of items, with the term "and" or "or" to separate any of the items, modifies the list as a whole, rather than each member of the list. The phrase "at least one of" does not require selection of at least one of each item listed; rather, the phrase allows a meaning that includes at a minimum one of any of the items, and/or at a minimum one of any combination of the items, and/or at a minimum one of each of the items. By way of example, the phrases "at least one of A, B, and C" or "at least one of A, B, or C" each refer to only A, only B, or only C; any combination of A, B, and C; and/or one or more of each of A, B, and C. Similarly, it may be appreciated that an order of elements presented for a conjunctive or disjunctive list provided herein should not be construed as limiting the disclosure to only that order provided.

One may appreciate that although many embodiments are disclosed above, that the operations and steps presented with respect to methods and techniques described herein are meant as exemplary and accordingly are not exhaustive. One may further appreciate that alternate step order or fewer or additional operations may be required or desired for particular embodiments.

Although the disclosure above is described in terms of various exemplary embodiments and implementations, it should be understood that the various features, aspects and functionality described in one or more of the individual embodiments are not limited in their applicability to the particular embodiment with which they are described, but instead can be applied, alone or in various combinations, to one or more of the some embodiments of the invention, whether or not such embodiments are described and whether or not such features are presented as being a part of a described embodiment. Thus, the breadth and scope of the present invention should not be limited by any of the above-described exemplary embodiments but is instead defined by the claims herein presented.

### ADDITIONAL NUMBERED STATEMENTS

1. An electronic watch comprising:
   a display assembly comprising:
      a display stack; and
      a cover positioned over the display stack, the cover defining a front surface of the electronic watch;
   a housing defining:
      an exterior side of the electronic watch;
      a recess along the exterior side;
      an internal volume; and
      a wall segment defining an end of the recess and fluidly isolating an external environment from the internal volume;
   a crown positioned along the exterior side of the electronic watch, the crown comprising:
      a cap configured to rotate in response to a rotational input;
      a shaft extending from the cap and positioned within the recess, the shaft configured to rotate in response to the rotational input; and
      a magnetic member positioned within the recess and coupled to the shaft, the magnetic member configured to rotate in response to the rotational input and to produce a change in a magnetic field that propagates through the wall segment; and
   a magnetic sensing system positioned within the internal volume and configured to detect the rotational input based at least in part on a change in the magnetic field due to rotation of the magnetic member.
2. The electronic watch of statement 1, wherein:
   the cap, the shaft, and the magnetic member are configured to translate towards the wall segment in response to a translational input; and
   the magnetic sensing system is configured to detect the translational input based at least in part on a change in the magnetic field due to the translation of the magnetic member.
3. The electronic watch of statement 1 or statement 2, further comprising:
   a dome switch coupled to the wall segment, the dome switch positioned within the recess and opposite the internal volume, the dome switch configured to bias the shaft away from the wall segment; and
   a conductive member positioned between the shaft and the dome switch, the conductive member conductively coupled to the wall segment.
4. The electronic watch of any preceding statement, wherein:
   the conductive member is conductively coupled to the cap;
   the cap defines a user input surface configured to receive an electrocardiogram (ECG) signal from a user; and
   the electronic watch further comprises a processing system conductively coupled to the wall segment and configured to detect the ECG signal via the wall segment.
5. The electronic watch of any preceding statement, wherein:
   the housing comprises:
      a main body defining an opening extending from the exterior side; and
      a bucket structure positioned within the opening and configured to fluidly isolate the external environment from the internal volume, the bucket structure defining the wall segment; and
   the recess is defined by the opening and the bucket structure.
6. The electronic watch of any preceding statement, wherein the wall segment is formed from titanium.
7. The electronic watch of statement 1 or statement 2, wherein the magnetic sensing system comprises:
   a dome switch coupled to the wall segment and configured to bias the shaft away from the wall segment;
   a first tunneling magnetoresistance (TMR) sensor positioned at a first orientation and within the internal volume opposite the dome switch;
   a second TMR sensor positioned at a second orientation different from the first orientation and within the internal volume opposite the dome switch; and
   a magnetic shielding member at least partially surrounding the first and second TMR sensors.
8. An electronic device comprising:
   a housing formed from a metal material, the housing comprising a sidewall and at least partially defining an internal volume of the electronic device;
   a crown comprising:
      a cap positioned outside of the internal volume and along an exterior side of the sidewall, the cap configured to rotate in response to a user input;
      a magnetic member coupled to the cap and positioned outside of the internal volume, the magnetic member configured to rotate in response to the user input; and
   a magnetic sensing system positioned within the internal volume of the electronic device and along an interior side of the sidewall opposite the exterior side, the magnetic sensing system configured to detect the user input based at least in part on a change in a magnetic field due to the rotation of the magnetic member.
9. The electronic device of statement 8, wherein:
   the housing further defines:
      an external portion;
      a hole extending through the external portion; and
      a secondary volume fluidly coupled to an external environment and fluidly isolated from the internal volume, the secondary volume defined by the sidewall and the external portion;
   the crown extends at least partially through the hole; and
   the magnetic member is positioned within the secondary volume.
10. The electronic device of statement 9, wherein the user input is a first user input and the electronic device further comprises:
   a dome switch positioned within the secondary volume and coupled to the sidewall, the dome switch configured to:
   bias the crown away from the sidewall; and
   collapse in response to a second user input.
11. The electronic device of any of statements 8 to 10, wherein:
   the housing comprises an external protrusion extending from the sidewall;
   the crown is coupled to the external protrusion; and
   the magnetic member is positioned radially outward of the external protrusion.
12. The electronic device of statement 11, wherein the external protrusion is configured to transmit an electrocardiogram (ECG) signal from a user input surface to a flexible circuit positioned within the internal volume.
13. The electronic device of any of statements 8 to 12, wherein:
   the electronic device further comprises a haptic actuator positioned within the internal volume of the electronic device; and
   the electronic device is configured to output a haptic response via the haptic actuator in response to the user input.
14. The electronic device of statement 13, further comprising a magnetic shielding member at least partially surrounding the magnetic sensing system, the magnetic shielding member configured to magnetically shield the magnetic sensing system from the haptic actuator.
15. An electronic device comprising:
   a housing comprising a metal sidewall, the metal sidewall defining:
      an exterior surface; and
      an interior surface opposite the exterior surface;
   a crown positioned along the exterior surface, the crown comprising:
      a cap configured to rotate in response to a rotational input; and
      a magnetic member configured to:
         rotate with the cap in response to the rotational input; and
         propagate a magnetic field through the metal sidewall; and
   a magnetic sensing system configured to detect, through the metal sidewall, a change in the magnetic field due to rotation of the magnetic member.
16. The electronic device of statement 15, wherein:
   the crown further comprises a shaft extending from the cap;
   the magnetic member is coupled to the shaft; and
   the magnetic member includes a north polarity and a south polarity, the north and south polarities radially opposed about a rotational axis of the crown.
17. The electronic device of statement 15 or statement 16, wherein:
   the crown comprises a rotating assembly configured to rotate in response to the rotational input;
   the rotating assembly comprises the cap and the magnetic member; and
   an entirety of the rotating assembly is external with respect to the exterior surface.
18. The electronic device of any of statements 15 to 17, wherein the magnetic sensing system comprises:
   a first tunneling magnetoresistance (TMR) sensor positioned at a first orientation along the interior surface; and
   a second TMR sensor positioned at a second orientation different from the first orientation, the second TMR sensor positioned along the interior surface, the magnetic sensing system configured to detect a direction of rotation of the rotational input.
19. The electronic device of any of statements 15 to 18, wherein:
   the magnetic member is a first magnetic member;
   the crown further comprises a second magnetic member; and
   the first and second magnetic members are positioned along a periphery of the cap.
20. The electronic device of any of statements 15 to 19, wherein the housing is a monolithic structure.

## Claims

1. An electronic device comprising:
a housing formed from a metal material, the housing comprising a sidewall and at least partially defining an internal volume of the electronic device;
a crown comprising:
a cap positioned outside of the internal volume and along an exterior side of the sidewall, the cap configured to rotate in response to a user input;
a magnetic member coupled to the cap and positioned outside of the internal volume, the magnetic member configured to rotate in response to the user input; and
a magnetic sensing system positioned within the internal volume of the electronic device and along an interior side of the sidewall opposite the exterior side, the magnetic sensing system configured to detect the user input based at least in part on a change in a magnetic field due to the rotation of the magnetic member.

2. The electronic device of claim 1, wherein:
the housing further defines:
an external portion;
a hole extending through the external portion; and
a secondary volume fluidly coupled to an external environment and fluidly isolated from the internal volume, the secondary volume defined by the sidewall and the external portion;
the crown extends at least partially through the hole; and
the magnetic member is positioned within the secondary volume.

3. The electronic device of claim 2, wherein the user input is a first user input and the electronic device further comprises:
a dome switch positioned within the secondary volume and coupled to the sidewall, the dome switch configured to:
bias the crown away from the sidewall; and
collapse in response to a second user input.

4. The electronic device of any preceding claim, wherein:
the housing comprises an external protrusion extending from the sidewall;
the crown is coupled to the external protrusion; and
the magnetic member is positioned radially outward of the external protrusion.

5. The electronic device of claim 4, wherein the external protrusion is configured to transmit an electrocardiogram, ECG, signal from a user input surface to a flexible circuit positioned within the internal volume.

6. The electronic device of any preceding claim, wherein:
the electronic device further comprises a haptic actuator positioned within the internal volume of the electronic device; and
the electronic device is configured to output a haptic response via the haptic actuator in response to the user input.

7. The electronic device of claim 6, further comprising a magnetic shielding member at least partially surrounding the magnetic sensing system, the magnetic shielding member configured to magnetically shield the magnetic sensing system from the haptic actuator.

8. The electronic device of claim 1, wherein:
the electronic device further comprises:
a display assembly comprising:
a display stack; and
a cover positioned over the display stack, the cover defining a front surface of the electronic device;
the sidewall further defines a recess along the exterior side;
the sidewall fluidly isolates an external environment from the internal volume;
the crown further comprises a shaft extending from the cap and positioned within the recess, the shaft configured to rotate in response to the user input; and
the magnetic member is positioned at least partially within the recess.

9. The electronic device of claim 8, wherein:
the cap, the shaft, and the magnetic member are configured to translate towards the sidewall in response to a translational input; and
the magnetic sensing system is configured to detect the translational input based at least in part on a change in the magnetic field due to the translation of the magnetic member.

10. The electronic device of claim 9, further comprising:
a dome switch coupled to the sidewall, the dome switch positioned within the recess and opposite the internal volume, the dome switch configured to bias the shaft away from the sidewall; and
a conductive member positioned between the shaft and the dome switch, the conductive member conductively coupled to the sidewall.

11. The electronic device of claim 10, wherein:
the conductive member is conductively coupled to the cap;
the cap defines a user input surface configured to receive an electrocardiogram, ECG, signal from a user; and
the electronic device further comprises a processing system conductively coupled to the sidewall and configured to detect the ECG signal via the sidewall.

12. The electronic device of any of claims 8 to 11, wherein:
the housing comprises:
a main body defining an opening extending from the exterior side; and
a bucket structure positioned within the opening and configured to fluidly isolate the external environment from the internal volume, the bucket structure defining the sidewall; and
the recess is defined by the opening and the bucket structure.

13. The electronic device of any of claims 8 to 12, wherein the sidewall is formed from titanium.

14. The electronic device of claim 8 or claim 9, wherein the magnetic sensing system comprises:
a dome switch coupled to the sidewall and configured to bias the shaft away from the sidewall;
a first tunneling magnetoresistance, TMR, sensor positioned at a first orientation and within the internal volume opposite the dome switch;
a second TMR sensor positioned at a second orientation different from the first orientation and within the internal volume opposite the dome switch; and
a magnetic shielding member at least partially surrounding the first and second TMR sensors.

15. The electronic device of any preceding claim, wherein the magnetic member includes a north polarity and a south polarity, the north and south polarities radially opposed about a rotational axis of the crown.
